Europäisches Patentamt

European Patent Office

Office européen des brevets

(19)

(11) Publication number: **0 185 197**
**B1**

# (12) EUROPEAN PATENT SPECIFICATION

(45) Date of publication of patent specification: **26.04.89**

(21) Application number: **85114420.4**

(22) Date of filing: **13.11.85**

(60) Divisional applications 88106055, 88106056 filed on 15.04.88.

(51) Int. Cl.⁴: **A 61 F 13/00**, A 61 F 13/10, A 61 F 13/12

(54) A fixation bandage.

(30) Priority: **21.11.84 DK 5528/84**

(43) Date of publication of application:
**25.06.86 Bulletin 86/26**

(45) Publication of the grant of the patent:
**26.04.89 Bulletin 89/17**

(84) Designated Contracting States:
**DE FR GB IT NL SE**

(56) References cited:
**CH-A- 436 548
DE-C- 570 884
DE-U-1 694 609
US-A-2 646 796
US-A-3 279 465
US-A-3 306 288
US-A-3 327 703**

(73) Proprietor: **Mölnlycke AB
S-405 03 Göteborg (SE)**

(73) Proprietor: **Tytex A/S
Industrivej 21
DK-7430 Ikast (DK)**

(72) Inventor: **Kristensen, Johannes Nyvang
Laesogade 3
DK-7430 Ikast (DK)**
Inventor: **Thygesen, Eskild Georg
Broholmvej 14
Tulstrup DK-7430 Ikast (DK)**
Inventor: **Grindsted, Erik
Frederiksberg Alle 19A, 4.th
DK-1820 Frederiksberg C. (DK)**
Inventor: **Lundell, Malte
Bokvägen 8
S-435 00 Mölnlycke (SE)**

(74) Representative: **Patentanwälte Leinweber &
Zimmermann
Rosental 7/II Aufg.
D-8000 München 2 (DE)**

Courier Press, Leamington Spa, England.

EP 0 185 197 B1

## Description

The present invention relates to a fixation bandage consisting of elastic material and intended to hold a wound dressing, such as a compress, firmly in position on a peripheral body portion of a . person, said fixation bandage being preferably made by knitting or crocheting and being shaped mainly as a hose or flexible tube in one piece, said hose or flexible tube comprising a holding portion for receiving the body portion considered, at least one fastening portion and at least one gap, said fastening portion forming a cuff, collar, loop or the like for embracing a joint adjacent to the body portion in question of a person, the fixation bandage being so shaped and having an elasticity such that both the holding portion and the fastening portion can be slipped over the body portion considered in such a manner as to have its fastening portion placed around the adjacent joint.

A fixation bandage of this type is disclosed in US—A—3 279 465. This prior fixation bandage is adapted to be placed on those parts of the human body, like the trunk and the abdomen. The bandage has the shape of a T-shirt and is made of a knitted fabric comprising inelastic yarns and elastic yarns which are arranged in a zig-zag configuration.

It is known to hold wound dressings in position by means of conventional bandages, particularly gauze bandages. However, this has the disadvantage that the correct holding of the wound dressing depends very much on the tightness with which the bandage has been applied so that there is a. risk that the holding of the wound dressing may become either too loose or too tight.

It is also known to hold wound dressings in position by means of elastic tubular meshed fabrics, which are intended to provide an improved holding of the dressing because they can expand and contract in all directions. Thereby the above mentioned drawback in respect of tightness of the bandage is eliminated. However, these tubular meshed fabrics cannot be used in connection with all types of dressings.

For the holding of finger dressings it is known to use articles of the gauze bandage type comprising an envelope portion which embraces the dressing and is provided with two straps which are laid along the back of the hand and are tied around the wrist to hold the dressing in position on the finger.

These articles have several drawbacks. Thus, they are apt to become unravelled when the strips are made by splitting the upper portion of the gauze bandage material. Moreover, it will be difficult for the injured person to fasten the bandage, because he has only one hand available for tying the knot around the wrist. Furthermore, these bandaging articles are apt to become loose owing to movements of the wrist so that they no longer secure a satisfactory holding of the dressing.

It is the object of the present invention to remedy the drawbacks mentioned above by providing a fixation bandage which is flexible and elastic and ensures the maintenance in position of the dressing on the finger even in the case of extreme movements thereof, and which is easy to apply whether it is done by the injured person himself or by a person assisting him for the purpose. To achieve this object the invention is characterized in that the hose or flexible tube is closed at one end in the holding portion and is open at its other end which is in the fastening portion, that the gap is provided in the side of the hose and extends substantially in the longitudinal direction of the hose and is located adjacent the fastening portion of the hose, and that, the fastening portion is adapted, after the holding portion has been applied to a finger in the form of a fingerstall, to be slipped over the finger and the whole of the hand to place the cuff around the wrist, or which is adapted, after the holding portion has been applied to a toe, to be slipped over the toe and the whole of the foot to place the cuff around the ankle joint and under the arch of the foot.

Further developments of the invention are set forth in the depending claims.

The invention will now be described in more detail with reference to an exemplifying, nonlimiting embodiment thereof illustrated in the accompanying drawings, in which

Fig. 1 is a diagrammatic illustration of a fixation bandage as applied to a forefinger, and

Fig. 2 is a diagrammatic side view of a knitted fixation bandage having two gaps.

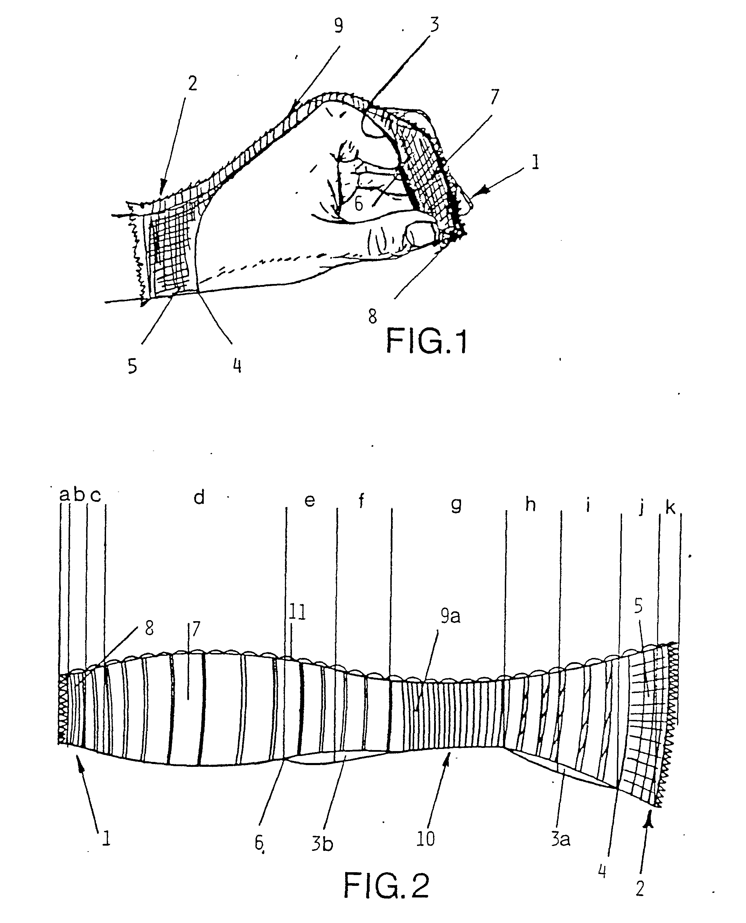

An embodiment of the fixation bandage according to the invention having a single gap will now be explained with reference to Fig. 1.

The fixation bandage, which is made from an elastic material, is provided in the form of an elongated hose which is closed at one end 1 and open at its other end 2. The hose is provided with at least one gap 3, which extends from a point 4 adjacent the open end 2 of the hose. Hereby a cuff is formed which is adapted to be placed around the wrist of the user. The gap 3 extends along the length of the hose to a point 6 which is located at a distance from the closed end 1 such that a fingerstall 7 is formed. The fingerstall has a length sufficient for embracing at least a substantial portion of the length of a finger. At its closed end 1 the fingerstall 7 is terminated by a cushion 8, which protects the injured person against thrust from outside in the longitudinal direction of the finger. The fixation bandage is so made that it has a smaller diameter at the closed end 1 and the fingerstall 7 and a larger diameter at the open end 2 and the cuff 5.

The cuff 5 and the fingerstall 7 are connected with each other by means of a portion of the hose present between the cuff and the fingerstall and forms a connecting string 9. This extends along the back of the hand and holds the fingerstall in position relatively to the wrist, even under extreme movements of the body portions considered.

The manner in which the fixation bandage is applied will now be described.

The user introduces the finger, to which a dressing has been applied, through the gap 3 into the fingerstall 7. This is a simple operation, because it is performed while the fixation bandage is in its non-stretched condition. Thereafter the whole of the hand, with the fingerstall 7 remaining on the finger, is introduced through the cuff 5 from the open end 2 of the hose and through the gap 3. Thereafter the cuff can readily be slipped inside out up around the wrist, whereby the connecting strin 9 is stretched and maintains the fingerstall in position.

Reference is now made to Fig. 2, which shows another embodiment which is knitted and has two gaps 3*a*, 3*b*. The fixation bandage according to this embodiment differs from that described above in that besides a first gap 3*a* adjacent to the open end 1 by an additional gap 3*b* is provided. The distance between the first gap 3*a* and the open end 1 of the hose substantially corresponds to the distance between the open end 2 and the gap 3 previously described, so that a cuff 5 substantially identical to that previously described is formed. The additional gap 3*b* is arranged between the first gap 3*a* and the closed end 1 such that a fingerstall is formed having a size as previously described. Thus, the two gaps are separated by an intermediate hose portion 10. In this embodiment a coloured thread 11 is shown, which serves as a code to indicate the size of the fixation bandage. It is contemplated to produce the fixation bandage in 3—4 different sizes in order to obtain optimum adaptation to different hand sizes.

To apply this fixation bandage the finger is first introduced through the additional gap 3*b* into the fingerstall 7. Thereafter the whole of the hand is introduced through the open end 2 and stuck out through the first gap 3*a*. Thereafter, the cuff 5 can easily be slipped up around the wrist in the same manner as described above. The connecting string 9*a*, which is formed in this embodiment, is narrower than the connecting string 9 of the embodiment first described. The connecting string 9*a* will thus be less conspicuous, and the risk of the user's hand to get caught at the narrower connecting string is reduced.

The fixation bandage of Fig. 2 is made with different types of knitting distributed over its length. As seen in succession from the closed end, the fixation bandage comprises

a zone *a* with fringes extending towards the next following fixation bandage in the knitting process,

a zone *b* which is tightly knitted with a small diameter to form the cushion 8,

a zone *c* which is firmly knitted at the end of the fingerstall 7,

a zone *d* with a more open knitting and a larger diameter,

a zone *e* with an open knitting along one half of the additional gap 3*b*,

a zone *f* with a less open knitting and with decreasing diameter along the second half of the additional gap 3*b*,

a zone *g* with a tight knitting and with a smaller diameter to form a narrow connecting string 9*a*,

zones *h, i, j* which are approximately conically knitted with the larger diameter towards the open end 2 and the cuff 5, and

a zone *k* with fringes extending towards a successive fixation bandage in the knitting process to provide for severing.

Though the fixation bandage for a finger dressing has been described with reference to two specific embodiments, the fixation bandage for a finger dressing can also be constructed otherwise, e.g. the fingerstall of the fixation bandage can be provided with a dressing so that the fixation bandage and the dressing are applied in one operation in the same manner as described above. Other fixation bandages can also be provided with a dressing for application in one operation.

No fixation bandages for toe dressings have been shown, but these could in principle be constructed in the same manner as the finger fixation bandage shown in Figs. 1 and 2, but with a gap for the heel provided in the fixing portion and adapted to be applied around the ankle joint and under the arch of the foot.

## Claims

1. A fixation bandage consisting of elastic material and intended to hold a wound dressing, such as a compress, firmly in position on a peripheral body portion of a person, said fixation bandage being preferably made by knitting or crocheting and being shaped mainly as a hose or flexible tube in one piece, said hose or flexible tube comprising a holding portion (7) for receiving the body portion considered, at least one fastening portion (5) and at least one gap (3, 3*a*, 3*b*), said fastening portion forming a cuff for embracing a joint adjacent the body portion in question of a person, the fixation bandage being so shaped and having an elasticity such that both the holding portion (7) and the fastening portion (5) can be slipped over the body portion considered in such a manner as to have its fastening portion placed around the adjacent joint, characterized in that the hose or flexible tube is closed at one end (1) in the holding portion (7) and is open at its other end (2) in the fastening portion (5), that the gap (3, 3*a*) is provided in the side of the hose and extends substantially in the longitudinal direction of the hose and is located adjacent the fastening end (5) of the hose, and that the fastening portion (5) is adapted, after the holding portion (7) has been applied to a finger in the form of a fingerstall, to be slipped over the finger and the whole of the hand to place the cuff around the wrist, or which is adapted, after the holding portion has been applied to a toe, to be slipped over the toe and the whole of the foot to place the cuff around the ankle joint and under the arch of the foot.

2. A fixation bandage as in claim 2, characterized in that an additional gap (3*b*) is provided in a position between the first gap (3*a*) and the closed end (1) of the hose.

3. A fixation bandage as in claim 2 or 3, characterized in that the hose has a smaller diameter at its closed end (1) and a larger diameter at its open end (2).

4. A fixation bandage as in any of the beforegoing claims, characterized in that the terminal portion of the closed end (1) form a cushion (8).

5. A fixation bandage as in any of the beforegoing claims, characterized in that the hose is made with different types of knitting (a—b) distributed over the length of the hose.

6. A fixation bandage as in any of the beforegoing claims, characterized in that the hose is provided with a colour code (11) to indicate size.

7. A fixation bandage as in any of the beforegoing claims, characterized in that the hose is made from two knitted or crocheted webs which are interconnected at their edges (12, 13) to form the hose.

**Patentansprüche**

1. Fixierungsbandage, die aus einem elastischen Material besteht und dazu dient, einen Wundverband, wie z.B. eine Kompresse, an einer Körperextremität einer Person festzulegen, wobei die Fixierungsbandage vorzugsweise durch Strikken oder Häkeln hergestellt und im wesentlichen einstückig in Form eines Schlauches oder flexiblen Rohres ausgebildet ist, wobei der Schlauch oder das flexible Rohr einen Haltebereich (7) zur Aufnahme des betreffenden Körperteils, mindestens einen Befestigungsbereich (5) und mindestens eine Lücke (3, 3a, 3b) umfaßt, daß der Befestigungsbereich eine Manschette bildet, um ein dem betreffenden Körperteil benachbartes Gelenk zu umfassen, wobei die Fixierungsbandage so geformt ist und eine solche Elastizität aufweist, daß sowohl der Haltebereich (7) als auch der Befestigungsbereich (5) derart auf den betreffenden Körperteil aufgestreift werden kann, daß ihr Befestigungsbereich das benachbarte Gelenk umgibt, dadurch gekennzeichnet, daß der Schlauch oder das flexible Rohr an einem Ende (1) im Haltebereich (7) geschlossen und an seinem im Befestigungsbereich (5) befindlichen anderen Ende (2) offen ist, daß die Lücke (3, 3a) auf der Seite des Schlauches ausgebildet ist und sich im wesentlichen in Längsrichtung des Schlauches erstreckt und neben dem Befestigungsbereich (5) des Schlauches angeordnet ist und daß der Befestigungsbereich (5) über einen Finger und die gesamte Hand übergestreift werden kann, um die Manschette um das Handgelenk herum anzuordnen, nachdem der Haltebereich (7) in der Art eines Fingerfutters auf dem Finger angeordnet wurde, oder daß der Befestigungsbereich über einen Zeh und den gesamten Fuß übergestreift werden kann, um die Manschette um das Fußgelenk herum und unter dem Fußgewölbe anzuordnen, nachdem der Haltebereich auf dem Zeh angeordnet wurde.

2. Fixierungsbandage nach Anspruch 1, dadurch gekennzeichnet, daß eine zusätzliche Lücke (3b) an einer Stelle zwischen der ersten Lücke (3a) und dem geschlossenen Ende (1) des Schlauches ausgebildet ist.

3. Fixierungsbandage nach Anspruch 1 oder 2, dadurch gekennzeichnet, daß der Schlauch einen kleineren Durchmesser an seinem geschlossenen Ende (1) und einen größeren Durchmesser an seinem offenen Ende (2) aufweist.

4. Fixierungsbandage nach einem der vorhergehenden Ansprüche, dadurch gekennzeichnet, daß der Endbereich des geschlossenen Endes (1) ein Polster (8) bildet.

5. Fixierungsbandage nach einem der vorhergehenden Ansprüche, dadurch gekennzeichnet, daß der Schlauch mit verschiedenen Strickmustern (A—B) hergestellt ist, die über die Länge des Schlauches verteilt sind.

6. Fixierungsbandage nach einem der vorhergehenden Ansprüche, dadurch gekennzeichnet, daß der Schlauch mit einer die Größe anzeigenden farbigen Markierung (11) versehen ist.

7. Fixierungsbandage nach einem der vorhergehenden Ansprüche, dadurch gekennzeichnet, daß der Schlauch aus zwei gestrickten oder gehäkelten Streifen hergestellt ist, die zur Bildung des Schlauches an ihren Rändern (12, 13) miteinander verbunden sind.

**Revendications**

1. Bandage de fixation consistant en un matériau élastique et destiné à maintenir un bandage pour blessure, tel qu'une compresse, fermement en position sur une partie périphérique du corps d'une personne, ledit bandage de fixation étant de préférence obtenu par tricotage ou crochetage et étant conformé principalement en un manchon ou tube flexible d'une pièce, ledit manchon ou tube flexible comprenant une partie de maintien (7) pour recevoir la partie du corps considérée, au moins une partie de fixation (5) et au moins une ouverture (3, 3a, 3b), ladite partie de fixation formant une manchette pour embrasser une articulation adjacente à la partie du corps en question d'une personne, le bandage de fixation étant conformé de telle manière et ayant une élasticité telle qu'à la fois la partie de maintien (7) et la partie de fixation (5) peuvent être glissées audessus de la partie du corps considérée de telle manière que sa partie de fixation soit placée autour de l'articulation adjacente, caractérisé en ce que le manchon ou tube flexible est fermé à une extrémité (1) dans la partie de maintien (7) et est ouvert à son autre extrémité (2) dans la partie de fixation (5), en ce que l'ouverture (3, 3a) est prévue dans le côté du manchon et s'étend sensiblement dans la direction longitudinale du manchon et est située de façon adjacente à l'extrémité de fixation (5) du manchon, et en ce que la partie de fixation (5) est adaptée, après que la partie de maintien (7) ait été appliquée à un doigt sous forme d'un doigtier, pour être glissée au-dessus du doigt et de l'ensemble de la main pour placer la manchette autour du poignet, ou qui est adaptée, après que la partie de maintien ait été appliquée à un orteil, pour être glissée au-dessus de l'orteil et de l'ensemble du pied pour placer la manchette autour de la cheville et sous la cambrure du pied.

2. Bandage de fixation selon la revendication 1, caractérisé en ce qu'une ouverture supplémentaire (3b) est prévue dans une position entre la première ouverture (3a) et l'extrémité fermée (1) du manchon.

3. Bandage de fixation selon la revendication 2 ou 3, caractérisé en ce que le manchon a un diamètre plus petit au niveau de son extrémité fermée (1) et un diamètre plus grand au niveau de son extrémité ouverte (2).

4. Bandage de fixation selon l'une des revendications précédentes, caractérisé en ce que la partie terminale de l'extrémité fermée (1) forme un coussin (8).

5. Bandage de fixation selon l'une des revendications précédentes, caractérisé en ce que le manchon est fait avec différents types de tricotage (a—b) distribués sur la longueur du manchon.

6. Bandage de fixation selon l'une des revendications précédentes, caractérisé en ce que le manchon est muni d'un code de couleur (11) pour indiquer la taille.

7. Bandage de fixation selon l'une des revendications précédentes, caractérisé en ce que le manchon est fait de deux tissus tricotés ou crochetés qui sont interreliés à leurs bords (12, 13) pour former le manchon.

FIG.1

FIG.2

1